# EUROPEAN PATENT SPECIFICATION

(11) **EP 3 733 205 B1**
(45) Date of publication and mention of the grant of the patent: **19.11.2025**
(21) Application number: 18895831.8
(22) Date of filing: 27.12.2018
(51) Int. Cl.: A61K 45/00, G01N 33/50, G01N 33/15, C12Q 1/6844, A61K 35/36, A61K 35/76, A61P 9/10, A61K 35/44, A61P 25/20, A61P 25/28, A61P 43/00

(54) **LIPOCALIN-TYPE PROSTAGLANDIN D2 SYNTHASE PRODUCTION ACCELERATING AGENT**
BESCHLEUNIGER DER PRODUKTION DER SYNTHASE VON LIPOCALIN-TYP-PROSTAGLANDIN-D2
AGENT D'ACCÉLÉRATION DE LA PRODUCTION DE PROSTAGLANDINE D2 SYNTHASE DE TYPE LIPOCALINE

(30) Priority: 28.12.2017 JP 2017253170
(43) Date of publication of application: 04.11.2020
(73) Proprietor: Hyogo College of Medicine, Hyogo 663-8501 (JP); Nippon Zoki Pharmaceutical Co., Ltd., Chuo-ku Osaka-shi, Osaka 541-0046 (JP)
(72) Inventor: MATSUYAMA, Tomohiro, Nishinomiya City, Hyogo 663-8501 (JP); NAKAGOMI, Takayuki, Nishinomiya City, Hyogo 663-8501 (JP); FUKUDA, Yu, Ono-shi, Hyogo 675-1363 (JP)
(74) Representative: Hoffmann Eitle
(86) International application number: PCT/JP2018/048141
(87) International publication number: WO 2019/131879

(56) References cited:
- WO-A1-00/43027
- WO-A1-2013/010170
- WO-A1-2018/161211
- JP-A- 2000 016 942
- JP-A- 2001 103 869
- JP-A- 2007 284 351
- JP-A- 2012 508 192
- JP-A- 2014 076 961
- JP-A- S6 479 657
- YAN Y ET AL: "Protection Mechanisms Against A beta 42 Aggregation", CURRENT ALZHEIMER RESEARCH, BENTHAM SCIENCE PUBL. LTD, NL, vol. 5, no. 6, 30 November 2008 (2008-11-30), pages 548 - 554, XP009530103, ISSN: 1567-2050, [retrieved on 20120301], DOI: 10.2174/156720508786898460
- MAESAKA JOHN K ET AL: "Prostaglandin D2 synthase: Apoptotic factor in alzheimer plasma, inducer of reactive oxygen species, inflammatory cytokines and dialysis dementia", vol. 2, no. 3, 30 June 2013 (2013-06-30), pages 166 - 180, XP009530106, ISSN: 2251-8363, Retrieved from the Internet <URL:https://www.ncbi.nlm.nih.gov/pmc/articles/PMC3891133/pdf/jnp-2-166.pdf> DOI: 10.12860/JNP.2013.28
- LESCUYER PIERRE ET AL: "Prostaglandin D2 synthase and its post-translational modifications in neurological disorders", ELECTROPHORESIS, VERLAG CHEMIE, vol. 26, no. 23, 30 November 2005 (2005-11-30), pages 4563 - 4570, XP009530104, ISSN: 0173-0835, [retrieved on 20051128], DOI: 10.1002/ELPS.200500292
- T. KANEKIYO ET AL: "Lipocalin-type prostaglandin D synthase/beta-trace is a major amyloid beta-chaperone in human cerebrospinal fluid", PROCEEDINGS OF THE NATIONAL ACADEMY OF SCIENCES, vol. 104, no. 15, 10 April 2007 (2007-04-10), pages 6412 - 6417, XP055622522, ISSN: 0027-8424, DOI: 10.1073/pnas.0701585104
- DATABASE WPI Week 200614, Derwent World Patents Index; AN 2006-136871, XP002804612
- MAR�A D GANFORNINA ET AL: "Molecular characterization and developmental expression pattern of the chicken apolipoprotein D gene: Implications for the evolution of vertebrate lipocalins", DEVELOPMENTAL DYNAMICS, WILEY-LISS INC NEW YORK , NY, US, vol. 232, no. 1, 3 December 2004 (2004-12-03), pages 191 - 199, XP071968488, ISSN: 1058-8388, DOI: 10.1002/DVDY.20193
- GARCIA-FERNANDEZ LF ET AL.: "Dexamethasone induces lipocalin-type prostaglandin D synthase gene expression in mouse neuronal cells", JOURNAL OF NEUROCHEMISTRY, vol. 75, no. 2, 2000, pages 460 - 470, XP55622513, ISSN: 0022-3042
- KIMURA, HIROSHI ET AL.: "A pilot study for clinical application of Neurotropin to senile patients with dementia", JAPANESE PHARMACOLOGY & THERAPEUTICS, vol. 15, no. 10, October 1987 (1987-10-01), pages 4235 - 4251, XP009521902, ISSN: 0386-3603
- SALEEM S ET AL.: "Lipocalin-prostaglandin D synthase is a critical beneficial factor in transient and permanent focal cerebral ischemia", NEUROSCIENCE, vol. 160, no. 1, 2009, pages 248 - 254, XP026036090, ISSN: 0306-4522, DOI: 10.1016/j.neuroscience.2009.02.039
- KANEKIYO T ET AL.: "Lipocalin-type prostaglandin D synthase/beta-trace is a major amyloid beta-chaperone in human cerebrospinal fluid", PROCEEDINGS OF THE NATIONAL ACADEMY OF SCIENCES OF THE UNITED STATES OF AMERICA, vol. 104, no. 15, 10 April 2007 (2007-04-10), pages 6412 - 6417, XP55622522, ISSN: 0027-8424

## Description

### [Technical Field]

The present invention is defined in the appended claims and uses a lipocalin-type prostaglandin D2 synthase (hereinafter, also referred to as "L-PGDS", and a synthase called "lipocalin-type prostaglandin D synthase" may be also included herein) production promoting agent.

### [Background Art]

It is known that prostaglandin D2 synthase (PGDS) includes two types. One type of the PGDS is lipocalin-type (L-)PGDS, which is distributed in the central nervous system, male genital organs, the heart or the like, and the other type is hematopoietic (H-)PGDS, which is distributed in mast cells and Th2 cells. L-PGDS has a catalytic activity for isomerization of prostaglandin H2 (PGH2), which is a common intermediate reaction in prostaglandin biosynthesis, to prostaglandin D2 (PGD2). On the other hand, L-PGDS structurally belongs to the lipocalin family, which acts as a carrier of a liposoluble substance. Thus, L-PGDS is a multifunctional protein acting as both a PGD2 biosynthetic enzyme and a carrier for lipophilic molecules.

L-PGDS distributed in the central nervous system is also detected in cerebrospinal fluid. L-PGDS has a huge lipophilic pocket as compared to other lipocalins. Thus, it is thought that L-PGDS acts as a transporter protein and a scavenger for various lipophilic molecules in the brain. Examples of the function of such L-PGDS as a lipocalin are as follows. For example, when subarachnoid hemorrhage occurs, an intracerebral L-PGDS level is increased, and protects brain from neuronal damages by conjugating with bilirubin, a neurotoxic substance (Inui T. et al., J. Cereb. Blood Flow Metab. 34, 1558-1567, 2014). Further, in Alzheimer's disease patients or animal models, L-PGDS strictly binds to a site essential for oligomerization of amyloid β-protein (Aβ, a senile plaque) to inhibit formation of Aβ deposition in cerebrospinal fluid and cytotoxicity (Kanekiyo T. et al., Proc. Natl. Acad. Sci. USA 104, 6412-6417, 2007).

In addition, it is reported that in the ischemic brain of mice, PGD2 production remarkably increases, and that gene deletion of L-PGDS and H-PGDS, or DP1, which is a PGD2 receptor, results in serious condition of cerebral edema occurred after ischemia (Tanigichi H. et al., J. Neurosci. 27, 4303-4312, 2007). It is also reported that knockout of L-PGDS results in extension of a cerebral infarction area or cerebral edema (Saleem S. et al., Neuroscience 160, 248-254, 2009). Accordingly, it is thought that PGD2 produced by L-PGDS and H-PGDS during cerebral ischemia acts so as to protect the brain via a receptor-mediated action.

Furthermore, it is reported that in a model mouse (the twitcher mouse) for Krabbe disease, which is a demyelinating disease caused by galactosylceramidase deficiency, in a demyelination-resistant axonal region, increase of expression of L-PGDS gene is observed. In addition, when L-PGDS gene of the model is further deleted, demyelination becomes more severe associating with disappearance of oligodendrocytes (Taniike M. et al., J. Neurosci. 22, 4885-4896, 2002). Further, it is reported that in an L-PGDS-knockout mouse, demyelination of a peripheral nerve is induced, and it is found that Gpr44, which is a PGD2 receptor in Schwann cells, is required for nerve myelination (Trimarco A. et al., Nature Neurosci. 17, 1682-1992, 2014). Based on these findings, it is thought that L-PGDS and PGD2 produced by L-PGDS are required for myelination and maintenance of the resulting myelin sheath of neuronal axons in oligodendrocytes (central nerve) and Schwann cells (peripheral nerve).

In addition, protective effects of L-PGDS are not limited to those on neuronal cells. With respect to the protective effects of L-PGDS, it is reported that cell deaths of glial cells in the gastrointestinal tract due to peroxidative stress can be avoided by 15-deoxyprostaglandin J2, which is a metabolite of PGD2 (Abdo H. et al., J. Physiol. 590, 2739-2750, 2012).

Furthermore, it has been known since a long time that intracerebral PGD2 synthesized by L-PGDS has a sleep-controlling action (Ueno R. et al., Biochem. Biophys. Res. Commun. 109, 576-582, 1982). The mechanism of the action has been examined in detail by Japanese investigators. As a result, it is said that PGD2 causes secretion of adenosine via a DP1 receptor on arachnoid mater at the base of the brain, and the adenosine stimulates the sleep center. It is assumed that since L-PGDS has a strong affinity to PGD2 as an enzymatic product, L-PGDS maintains stability of PGD2 in cerebrospinal fluid and transports PGD2 to a neighboring receptor (Urade Y. and Hayaishi O., Biochim. Biophys. Acta 1482, 259-271, 2000). Thus, it is thought that L-PGDS substantially contributes to sleep control via PGD2.

As described above, it is thought that L-PGDS is a protein that acts as an enzyme protein to catalyze the synthesis of PGD2, plays roles as a carrier/transporter and a scavenger for various hydrophobic low-molecular weight substances in the brain, and has various functions such as an intracerebral environment-controlling and brain-protective function, a sleep-controlling function, and the like. Thus, it is thought that when production of L-PGDS is promoted, these functions are effectively performed, which is useful for prevention/treatment of L-PGDS associated diseases . However, there is no report at present about a substance that promotes L-PGDS production in vivo.

In the present invention, it has been found that an extract from inflamed tissues inoculated with vaccinia virus (the present extract) has an excellent L-PGDS production promoting action. It is known that the present extract or a preparation containing the present extract exerts an extremely wide variety of actions and effects. For example, as an action and effect of the present extract on the brain, a therapeutic effect on an ischemic disease such as cerebral infarction (Japanese Patent Laid-Open No. 2000-16942), and an promoting action on production of neurotrophic factors such as BDNF (International Patent Application Publication No. WO 2011/162317). However, it is not known at present that the present extract has an L-PGDS production promoting action.

### [Summary of the Invention]

### [Problem to be Solved by the Invention]

Disclosed is a substance that promotes production of L-PGDS having a brain-protective action, a sleep-promoting action, and the like, and a screening system for a drug having a brain-protective action, a sleep-promoting action, and the like based on an L-PGDS production promoting action as an index, or the like. Also disclosed is an effective and highly safe pharmaceutical, which contains the above-described substance as an active ingredient, for the prevention/treatment or relapse prevention of L-PGDS associated diseases including a cerebrovascular disorder such as cerebral infarction, dementia such as Alzheimer's disease, insomnia, and the like. In addition, the L-PGDS production promoting agent has effects of suppressing and alleviating ischemic disorder, neuronal cell injury, and the like of the brain in a cerebrovascular disorder, or the like. It should be noted that the term "treatment" or "therapeutic" as used herein include the meanings of "alleviation", "improvement", "inhibition of progression", and the like. It should be also noted that a drug having a sleep-promoting action is sometimes referred to as a "hypnotic" and the term "hypnotic" as used herein may encompass a "sleeping drug", "sleep aid", "sleep-inducing agent", and "hypnotic agent".

### [Means for Solving the Problems]

The present inventors have made investigations using a mouse model of cerebral infarction (permanent middle cerebral artery occlusion model), and have found stem cells which are capable of differentiate into neural cells such as neuronal cells, astrocytes, and oligodendrocytes, and also other various cells in an infarct region in which mature neuron are dying by occlusion of blood stream. The stem cells have been designated as ischemia-induced multipotent stem cells (iSCs). Nestin, which is a neural stem cell marker and is highly expressed in the iSCs, is histochemically distributed in perivascular region from cranial pia mater to the cerebral cortex parenchyma, and the iSCs express vascular perivascular cell (pericyte) markers such as platelet-derived growth factor receptor β (PDGFRβ), neuron-glial antigen 2 (NG2), and the like. Thus, it is thought that the iSCs are derived from pericytes distributed in perivascular regions. It is thought that the pericytes constitute a neurovascular unit (NVU), which is a functionally and structurally basic unit of the brain, together with neurons, astrocytes, and vascular endothelial cells, and play important roles such as formation and maintenance of the blood-brain barrier, control of neural functions, and participation in the glymphatic system. It is demonstrated that iSCs are produced by reprogramming of pericytes at the time of brain injuries such as ischemia, and the iSCs are differentiated into neural cells by microenvironment created by vascular endothelial cells and the like. Thus, it is thought that the iSCs are stem cells that play a leading role in neural restoration along with vascular reconstruction after stroke.

The present inventors have made studies on effects of an extract from inflamed skins of rabbits inoculated with vaccinia virus (the present extract) on iSCs as a part of investigations of iSCs. The present inventors added the present extract to iSCs, cultured the iSCs, and then performed comprehensive gene expression analysis using a DNA chip for about 28,000 genes. As a result, the present inventors have found that the present extract has an activity to selectively promote expression of PTGDS, which is a gene encoding L-PGDS. In addition, it is confirmed that, with respect to protein level expression, the present extract promotes the production of L-PGDS. Further, by a result of an investigation using an immunohistochemistry technique, it is shown that L-PGDS expressed in an ischemic brain is co-localized with a pericyte marker. Thus, it is thought that L-PGDS is derived from pericytes or iSCs dedifferentiated from pericytes.

As described above, it is thought that L-PGDS is a protein that acts as an enzyme protein to catalyze the synthesis of PGD2, is mainly expressed in the brain, plays a role as a carrier/transporter and a scavenger for various hydrophobic low-molecular weight substances, and has various functions such as an intracerebral environment-controlling and brain-protective function, a sleep-controlling function, and the like. Thus, it is thought that an L-PGDS production promoting agent is useful as a preventive, therapeutic or relapse preventive agent for L-PGDS associated diseases, including cerebrovascular disorder such as cerebral infarction, dementia such as Alzheimer's disease, and insomnia. It has been confirmed that the present extract has an excellent L-PGDS production promoting action, increases the amount of L-PGDS and reduces the amount of Aβ in the brain of an Alzheimer disease model mouse, and thus improves cognitive functions. Accordingly, the present extract or a substance having an L-PGDS production promoting action contained in the present extract, or a preparation containing the present extract is extremely useful as an L-PGDS production promoting agent.

The present inventors also have found that an L-PGDS production promoting action in iSCs is useful as an index used in a screening method for a drug useful in the treatment of L-PGDS associated diseases as defined in the appended claims. It it thought that the screening method particularly contributes to the development of a drug having a brain-protective action or a sleep-promoting action. In addition, the present invention provides a method for determining and evaluating the action and effect of the present extract or a preparation containing the present extract by testing the present extract or the preparation containing the present extract using an L-PGDS production promoting action in iSCs as an index, and thus verifying drug efficacy of the present extract or the preparation containing the present extract as defined in the appended claims. The present inventors have accomplished the present invention based on the above findings.

That is, the invention of the present application embraces the following aspects, for example.

[Item 1] A screening method for a substance having a brain-protective action or a sleep-promoting action, comprising the steps of screening substances using a lipocalin-type prostaglandin D2 synthase expression-promoting action in pericytes or ischemia-induced multipotent stem cells dedifferentiated from pericytes as an index, and selecting a substance as having a brain-protective action or a sleep-promoting action based on the lipocalin-type prostaglandin D2 synthase expression-promoting action.

[Item 2] The screening method according to item 1, wherein the substance having a brain-protective action is for prevention, treatment or relapse prevention of a cerebrovascular disorder, or for prevention or treatment of dementia.

[Item 3] A determining or evaluating method for an extract from inflamed skin tissues of rabbits inoculated with vaccinia virus or a preparation containing the extract, comprising the steps of providing an extract from inflamed skin tissues of rabbits inoculated with vaccinia virus or a preparation containing the extract and determining or evaluating that the extract or the preparation is for the protection of brain or the sleep promotion using a lipocalin-type prostaglandin D2 synthase expression-promoting action as an index, optionally, wherein a lipocalin-type prostaglandin D2 synthase expression-promoting action in pericytes or ischemia-induced multipotent stem cells dedifferentiated from pericytes is used as an index.

[Item 4] A method for verifying that an extract from inflamed skin tissues of rabbits inoculated with vaccinia virus or a preparation containing the extract satisfies a quality standard by performing the determination or evaluation as described in item 3.

### [Advantages of the Invention]

It is strongly expected that an L-PGDS production promoting agent enhances a function of L-PGDS, which is expressed in pericytes or iSCs, as lipocalin, and the resulting L-PGDS acts as a transporter which causes excretion of various hydrophobic molecules from the brain, which in turn protects the brain from a harmful influence of an injury in cerebral ischemia or a substance which is thought to lead to dementia. In addition, it is expected that the L-PGDS production promoting agent causes PGD2 synthesized in a cell to be secreted into cerebrospinal fluid and transports the PGD2 to a PGD2 receptor in the brain, which leads to a sleep-controlling action, and the like. In particular, it has been found that the present extract promotes L-PGDS production in ischemic brain of a mouse. In addition, it is demonstrated that when the present extract is administered to an Alzheimer-type dementia model mouse, the amount of the L-PGDS in the brain is increased and the amount of Aβ is decreased, and cognitive function is improved. As described above, it is confirmed, even by experiments on animals, that the present extract promotes L-PGDS production and exerts an excellent pharmacological action. Further, a preparation containing the present extract has been used as a highly safe drug with little problem such as side effects for a long time. Thus, the present invention as defined in the appended claims is exceptionally useful.

In addition, the present extract is a multicomponent substance including an extremely enormous number of components. Thus, it is extremely difficult to determine or evaluate actions of the present extract by the mount of a single or plurality of components or the like, and verify drug efficacy of the present extract. On the other hand, according to a determination or evaluation method for the present extract or a preparation containing the present extract using an L-PGDS expression-promoting action as an index, the actions of the present extract or a preparation containing the present extract can be easily determined or evaluated, which in turn can be used for verifying drug efficacy of the present extract or a preparation containing the present extract. From these points, the present invention as defined in the appended claims is also highly useful. Herein, the term "determination or evaluation" encompasses all concepts for examining a target material by a test, an inspection, or the like to identify effects, actions, suitability, or the like of the test material.

### [Brief Description of Drawings]

Fig. 1 is a graph showing L-PGDS gene (PTGDS) expression level, as examined using real-time RT-PCR, in cultured iSCs to which a test substance was added.
Fig. 2 is an electropherogram showing L-PGDS gene (PTGDS) expression level, as examined using classical RT-PCR, in cultured iSCs to which a test substance was added.
Fig. 3 is an electropherogram showing L-PGDS protein expression level, as examined using Western blotting, in cultured iSCs to which a test substance was added.
Fig. 4 is a PVDF membrane showing L-PGDS protein expression level, as examined using dot blotting, in cultured iSCs to which a test substance was added.
Fig. 5 is a graph showing L-PGDS protein expression level, as examined using an ELISA method, in cultured iSCs to which a test substance was added.
Fig. 6 is a graph showing the amounts of prostaglandins, as examined using liquid chromatography-mass spectrometry, in a cell extract of cultured iSCs to which a test substance was added.
Fig. 7 is a graph showing the amounts of reaction products, as examined using liquid chromatography-mass spectrometry, after adding substrates for L-PGDS to a culture supernatant of cultured iSCs to which a test substance was added.
Fig. 8 represents images showing distributions of L-PGDS, pericytes, and vascular endothelial cells, as compared with each other using immunohistochemical staining, in the mouse brain to which ischemic insult was applied.
Fig. 9 represents images showing distributions of L-PGDS, as observed using immunoelectron microscopy, in the mouse brain to which ischemic insult was applied.
Fig. 10 represents images showing distributions of L-PGDS and nestin, which is a neural stem cell marker, as compared using immunohistochemical staining, in cultured iSCs isolated from a human cerebral infarction area.
Fig. 11 represents images showing deposition of amyloid β, as examined using immunohistochemical staining, in the brain of an Alzheimer-type dementia model mouse to which a test substance was administered.
Fig. 12 represents images showing the results of immunohistochemical staining of L-PGDS in the brain of an Alzheimer-type dementia model mouse to which a test substance was administered.
Fig. 13 represents comparative images showing distributions of L-PGDS and vascular endothelial cells, using immunohistochemical staining, in the brain of an Alzheimer-type dementia model mouse to which a test substance was administered.
Fig. 14 represents electropherograms showing expression levels of amyloid β and L-PGDS protein, as examined using Western blotting, in the brain of an Alzheimer disease model mouse to which a test substance was administered.
Fig. 15 represents electropherograms showing L-PGDS gene (PTGDS) expression levels, as examined using classical RT-PCR, in cultured pericytes to which a test substance was added.

### [Mode for Carrying Out the Invention]

The present extract is an extract containing a non-protein active substance extracted and separated from inflamed tissues of an animal having developed pox by being inoculated with vaccinia virus. The present extract is in liquid when it is extracted; however, the present extract may be made solid by drying. The present preparation is very useful as pharmaceuticals. One specific product that is manufactured and sold in Japan by the applicant as the present preparation is "Preparation containing an extract from inflamed skins of rabbits inoculated with vaccinia virus" (trade name: NEUROTROPIN [registered trademark]) (hereinafter, referred to as "NEUROTROPIN"). NEUROTROPIN includes injections and tablets, both of which are ethical drugs.

Indications of NEUROTROPIN injection are "low back pain, cervicobrachial syndrome, symptomatic neuralgia, itchiness accompanied by skin diseases (eczema, dermatitis, urticaria), allergic rhinitis and sequelae of subacute myelo-optico-neuropathy (SMON) such as coldness, paresthesia and pain". Indications of NEUROTROPIN tablet are "postherpetic neuralgia, low back pain, cervicobrachial syndrome, periarthritis scapulohumeralis and osteoarthritis". Present preparation has been created by the applicant and developed as a drug, and has been appreciated for its excellent advantage for efficacy and safety, sold for many years and established a firm position in the Japanese pharmaceutical market.

The extract from inflamed tissues inoculated with vaccinia virus used in the present invention can be obtained by the following manner: inflamed tissues inflamed by the inoculation with vaccinia virus is crushed; an extraction solvent is added to remove the tissue fragments; then deproteinization is carried out; the deproteinized solution is adsorbed onto an adsorbent; and then the active ingredient is eluted. for example, according to the following process.
(A) Inflamed skin tissues of rabbits, mice or the like by the inoculation with vaccinia virus are collected, and the inflamed tissues are crushed. To the crushed tissue an extraction solvent such as water, phenolated water, physiological saline or phenol-added glycerin water is added. Then, the mixture is filtered or centrifuged to obtain an extraction liquid (filtrate or supernatant).
(B) The pH of the extraction liquid is adjusted to be acidic and the liquid is heated for deproteinization. Then, the deproteinized solution is adjusted to be alkaline, heated, and then filtered or centrifuged.
(C) The obtained filtrate or supernatant is made acidic and adsorbed onto an adsorbent such as activated carbon or kaolin.
(D) To the adsorbent, an extraction solvent such as water is added, the pH is adjusted to alkaline, and the adsorbed component is eluted to obtain the extract from inflamed tissues inoculated with vaccinia virus. Subsequently, as desired, the eluate may be evaporated to dryness under reduced pressure or freeze-dried to give a dried material.

As for animals in order to obtain the inflamed tissues by the inoculation of vaccinia virus, various animals that is infected with vaccinia virus such as rabbits, cows, horses, sheep, goats, monkeys, rats or mice can be used, and preferred inflamed tissues are inflamed skin tissues of rabbits. With regard to a rabbit, any rabbit may be used so far as it belongs to Lagomorpha. Examples thereof include Oryctolagus cuniculus, domestic rabbit (domesticated Oryctolagus cuniculus), hare (Japanese hare), mouse hare and snowshoe hare. Among them, it is appropriate to use domestic rabbit. In Japan, there is family rabbit called "Kato" which has been bred since old time and frequently used as livestock or experimental animal and it is another name of domestic rabbit. There are many breeds in domestic rabbit and the breeds being called Japanese white and New Zealand white are advantageously used.

Vaccinia virus used herein may be in any strain. Examples thereof include Lister strain, Dairen strain, Ikeda strain, EM-63 strain and New York City Board of Health strain.

As to basic extracting steps (A) to (D) of the above-described for the present extract can be carried out in more detail, the following steps are used for example.

### About step (A):

The inflamed skin tissues of rabbits by the intradermal inoculation of vaccinia virus are collected. The collected skin tissues are washed and disinfected using a phenol solution, etc. This inflamed skin tissues are crushed and an extraction solvent in 1- to 5-fold thereof by volume is added thereto. Here, the term "crush" means to finely break down into minces using a mincing machine or the like. As to the extraction solvent, there may be used distilled water, physiological saline, weakly acidic to weakly basic buffer, etc. and bactericidal/antiseptic agent such as phenol, stabilizer such as glycerin, salts such as sodium chloride, potassium chloride or magnesium chloride, etc. may be appropriately added thereto. At that time, it is also possible that the cell tissue is destroyed by a treatment such as freezing/melting, ultrasonic wave, cell membrane dissolving enzyme or surfactant so as to make the extraction easier. The resulting suspension is allowed to stand for 5 to 12 days. During that period, the suspension may be heated at 30 to 45°C with or without appropriate stirring. The resulting liquid is subjected to a treatment for separating into solid and liquid (filtered or centrifuged, etc.) to remove the tissue fragments whereupon a crude extract (filtrate or supernatant) is obtained.

### About step (B)

The crude extract obtained in step (A) is subjected to a deproteinizing treatment. The deproteinization may be carried out by a known method which has been usually conducted and a method such as heating treatment, treatment with a protein denaturant (such as acid, base, urea, guanidine or an organic solvent including acetone), isoelectric precipitation or salting-out may be applied. After that, a common method for the removal of insoluble matters such as filtration using filter paper (such as cellulose or nitrocellulose), glass filter, Celite or Seitz filter, ultrafiltration or centrifugation is conducted to give a filtrate or a supernatant wherefrom the separated insoluble protein is removed.

### About step (C)

The filtrate or supernatant obtained in step (B) is adjusted to acidic or, preferably, to pH 3.5 to 5.5 to conduct an operation of adsorbing with an adsorbent. Examples of the usable adsorbent include activated carbon and kaolin. An adsorbent is added to the extract followed by stirring or the extract is passed through a column filled with an adsorbent so that the active ingredient can be adsorbed with the adsorbent. When an adsorbent is added to the extract, the adsorbent with which the active ingredient is adsorbed can be obtained by means of filtration, centrifugation, etc. to remove the solution.

### About step (D)

For elution (desorption) of the active ingredient from the adsorbent obtained in step (C), an elution solvent is added to said adsorbent and adjusted to basic or, preferably, to pH 9 to 12, elution is conducted at room temperature or with suitable heating, or with stirring, and then the adsorbent is removed by a common method such as filtration or centrifugation. As to the extraction solvent used therefore, there may be used a basic solvent such as water, methanol, ethanol, isopropanol or the like adjusted to basic pH or an appropriate mixed solvent thereof and preferably, water adjusted to pH 9 to 12 may be used. Amount of the extracting solvent may be appropriately set. In order to use the eluate obtained as such as a drug substance, the pH is appropriately adjusted to nearly neutral or the like whereby an extract from inflamed skins of rabbits inoculated with vaccinia virus (the present extract) can be finally obtained.

Since the present extract is liquid at the stage of being prepared, it is also possible that said extract is appropriately concentrated or diluted to make into a desired concentration. When a preparation is manufactured from the present extract, it is preferred to apply a sterilizing treatment with heating. For making into an injectable preparation, it is possible to add sodium chloride or the like so as to prepare a solution being isotonic to physiological saline. It is also possible that the present extract is administered in a liquid or gel state. Furthermore, the present extract may be subjected to an appropriate operation such as concentration to dryness to prepare a solid preparation for oral administration such as a tablet. Specific methods for the manufacture of solid preparation for oral administration from the present extract are disclosed in the specifications of Japanese Patent Nos. 3,818,657 and 4,883,798. The present preparation includes an injectable preparation, a solid preparation for oral administration, etc. prepared as such.

The administering method to a patient is not particularly limited and may be suitably selected depending on the purpose of treatment. Examples of the method include oral administration, subcutaneous administration, intramuscular administration, intravenous administration, and transdermal administration. The dose may be suitably determined depending on the type of the extract from inflamed tissues inoculated with vaccinia virus. The dose that is approved in the commercially available preparation is principally 16 NU per day by oral administration and 3.6 to 7.2 NU per day by injection. However, the dose may be appropriately increased or decreased depending on the type of disease, degree of seriousness, individual difference in the patients, method of administration, period of administration and the like (NU: Neurotropin unit. Neurotropin unit is defined by ED50 value of analgesic effect measured by a modified Randall-Selitto method using SART-stressed mice that are chronic stressed animals showing a lowered pain threshold than normal animals. One NU indicates the activity of 1 mg of analgesic ingredients in Neurotropin preparations when the ED50 value is 100 mg/kg of the preparation).

Hereinafter, examples of methods for producing the present extract as well as a novel pharmacological action of and results of pharmacological tests regarding a L-PGDS production promoting action and a peripheral nerve regeneration promoting action of the present extract are described.

### [Examples]

### Example 1 (Manufacture of the present extract)

Skins of healthy adult rabbits were inoculated with vaccinia virus intradermally and the inflamed skins were cut and collected. The collected skins were washed and disinfected by a phenol solution, an excessive phenol solution was removed and the residue was crushed. A phenol solution was added thereto and mixed therewith and the mixture was allowed to stand for 3 to 7 days, and further heated at 35 to 40°C together with stirring for 3 to 4 days. After that, an extracted solution obtained by a solid-liquid separation was adjusted to pH 4.5 to 5.2 with hydrochloric acid, heated at 90 to 100°C for 30 minutes and filtered to remove protein. The filtrate was adjusted to pH 9.0 to 9.5 with sodium hydroxide, heated at 90 to 100°C for 15 minutes and subjected to a solid-liquid separation.

The resulting deproteinized solution was adjusted to pH 4.0 to 4.3 with hydrochloric acid, activated carbon in an amount of 2% to the mass of the deproteinized solution was added thereto and the mixture was stirred for 2 hours and subjected to the solid-liquid separation. Water was added to the collected activated carbon followed by adjusting to pH 9.5 to 10 with sodium hydroxide and the mixture was stirred at 60°C for 90 to 100 minutes and centrifuged to give a supernatant. Water was added again to the activated carbon precipitated upon the centrifugation followed by adjusting to pH 10.5 to 11 with sodium hydroxide and the mixture was stirred at 60°C for 90 to 100 minutes and centrifuged to give a supernatant. Both supernatants were combined and neutralized with hydrochloric acid to give the present extract.

### Example 2: Pharmacological Test

Next, test methods and test results of pharmacological tests regarding an L-PGDS production promoting action using the present extract obtained in Example 1 as a test substance. Herein, in the following pharmacological tests, introduction of cerebral infarction in a C.B-17 mouse, and isolation and culture of iSCs obtained from the cerebral infarction area were performed according to methods described in Nakagomi, T. et al. Eur. J. Neurosci., 29, 1842-1852, 2009.

### Test Example 1: Comprehensive Gene Expression Analysis in iSCs Treated with Test Substance

Ischemic insult was applied to C.B-17 mice (3 animals) by middle cerebral artery occlusion, and 3 sets of cultured iSCs were isolated from infarction area at day 3. To each of the cultured iSCs (Dulbecco's Modified Eagle's Medium F12 (2% FBS DMEM/F12 F/E/N) supplemented with 2% fetal bovine serum (FBS), 20 ng/mL of fibroblast growth factor (FBS), 20 ng/mL of epidermal growth factor (EGF), and 1% of N2 Supplement, 5 × 10⁴ cell/3 cm φ dish), a test substance (50, or 1000 mNU/mL) or physiological saline (control) was added. Then, total RNA was extracted from the cultured iSCs by RNeasy [registered trademark, the same applies hereinafter] Mini Kit (QIAGEN) at day 4 of culture (9 cultures in total). For comprehensive gene expression analysis, SurePrint G3 Mouse GE Microarray 8×60K (24,321 kinds of RNAs and 4,576 kinds of noncoding RNAs, GE), which was a gene chip for mouse, was used. Then, a gene showing changes in expression to be 1/2 or less, in all of the 3 cultures, at the both concentrations of the test substance added, and a gene showing changes in expression to be twice or more, in all of the 3 cultures, at the both concentrations of the test substance added were selected. The results are shown by the ratio of expression levels of each gene selected above relative to those in the control (mean ± standard error of the three cultures). An example of the results is shown in Table 1.

**[Table 1]**

| Gene Name | Protein Name | Ratio of Expression Level | |
|---|---|---|---|
| | | 50 mNU/mL | 1000 mNU/mL |
| COCH | Coagulation factor C homolog | 0.40±0.01 | 0.31±0.03 |
| GBP6 | EGF-like domain, multiple 6 | 2.25±0.07 | 2.82±0.24 |
| PTGDS | L-type Prostaglandin D2 synthase | 4.57±0.83 | 48.42±5.58 |

From the results of the above analysis, as shown in Table 1, 3 genes, which were COCH gene with decreased expression and GBP6 gene and PTGDS gene with increased expression, were selected in total. Among these, the expression level of PTGDS gene encoding L-PGDS was strongly dependent on dose of the test substance.

### Test Example 2-1: Evaluation of L-PGDS Gene Expression (real-time RT-PCR method)

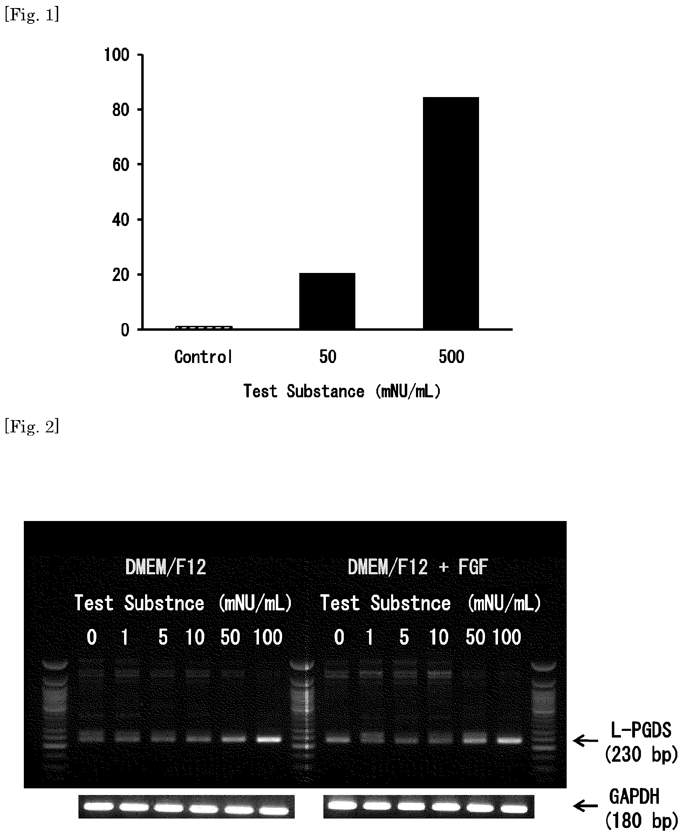

As in Test Example 1, to cultured iSCs (FBS-free DMEM/F12 F/-/-, 5 × 10⁴ cell/3 cm φ dish), a test substance (50, or 500 mNU/mL) or physiological saline (control) was added (n = 1), and the iSCs were cultured for 7 days. Then, RNA was extracted by Isogen II [registered trademark] (NIPPON GENE CO., LTD.) according to manufacturer's instructions. Purities of the RNA were determined on the basis of absorbance at 260 nm and 280 nm. The RNA was subjected to reverse transcription reaction using SuperScript [registered trademark, the same applies hereinafter] IV RTase (Invitrogen) in the presence of random primers to obtain single-stranded cDNAs corresponding to the total RNA. With respect to the resulting cDNAs, the amounts of transcription products were determined by quantitative PCR (Prism [registered trademark] 7900HT, Applied Biosystems) with PTGDS specific primers using β-Actin, which is a housekeeping gene, as a control (comparative threshold cycle method). The nucleotide sequences of the primers used for PTGDS and β-Actin were as follows: PTGDS: 5'-gactctgaaggacgagctgaag-3' (SEQ ID NO: 1) and 5'-tcttgaatgcacttatccggttgg-3' (SEQ ID NO: 2), β-Actin: 5'-tacagcttcaccaccacagc-3' (SEQ ID NO: 3) and 5'-aaggaaggctggaaaagagc-3' (SEQ ID NO: 4). The results are shown by the ratio of expression levels of PTGDS relative to that of β-Actin used as a control as 1. An example of the results is shown in Table 2 and Fig. 1.

**[Table 2]**

| Additive Concentration of Test Substance (mNU/mL) | Ratio of Expression Level of PTGDS to β-Actin (times) |
|---|---|
| 0 (Control) | 1.0 |
| 50 | 20.4 |
| 500 | 84.4 |

As apparent from Table 2 and Fig. 1, as confirmed by the results in Test Example 1, it was confirmed that expression of L-PGDS gene (PTGDS) in iSCs was promoted by the test substance in a dose dependent manner.

### Test Example 2-2: Evaluation of L-PGDS gene expression (classical RT-PCR method)

In the presence of a test substance each having a dose of 0 (control; physiological saline), 1, 5, 50, or 100 mNU/mL, iSCs were cultured in a medium containing FGF (DMEM/F12 F/-/-) or FGF-free medium (DMEM/F12 -/-/-) for 4 days. From the cultured iSCs, total RNA was extracted using RNeasy Mini Kit (QIAGEN), and L-PGDS gene (PTGDS) expression level was semiquantified by classical RT-PCR method using SuperScript III One-Step RT-PCR System with Platinum (Invitrogen) (35 cycles). The PCR products were separated by 2% agarose gel electrophoresis, and bands of PTGDS and GAPDH were stained with ethidium bromide and visually detected. The nucleotide sequences of the primers used for PTGDS and GAPDH were as follows: PTGDS: 5'-cctccaactcaagctggttc-3' (SEQ ID NO: 5) and 5'-atagttggcctccaccactg-3' (SEQ ID NO: 6), and GAPDH: 5'-atcactgccacccagaagac-3' (SEQ ID NO: 7) and 5'-cacattgggggtaggaacac-3' (SEQ ID NO: 8). An example of the results is shown in Fig. 2.

When L-PGDS gene (PTGDS) expression-promoting actions in iSCs were examined using varying doses, with smaller differences from one another, of the test substance, as shown in Fig. 2, L-PGDS gene expression level was promoted by the test substance in a dose dependent manner independent of whether the medium contains FGF or not.

### Test Example 3-1: Evaluation of L-PGDS protein Expression (western blotting method)

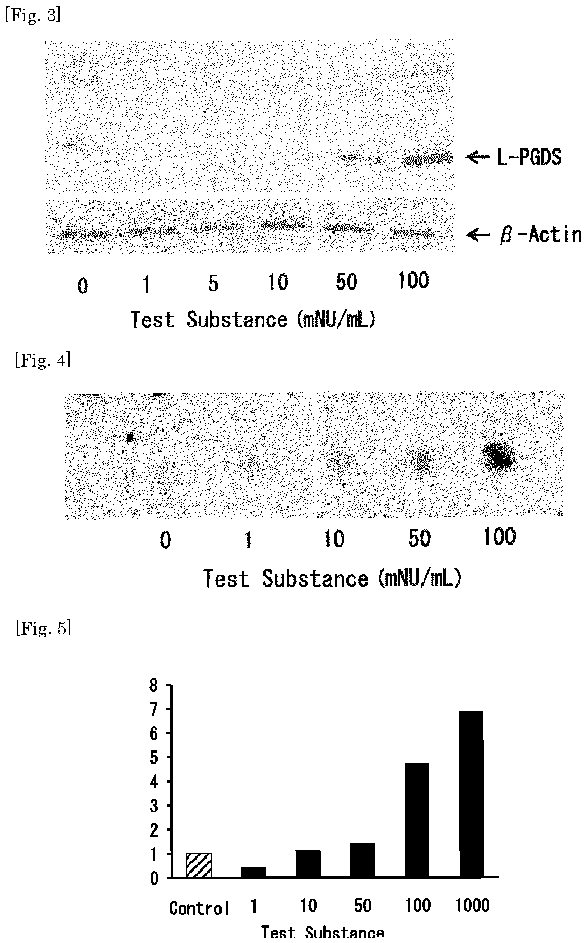

A test substance (0, 1, 5, 50, or 100 mNU/mL) was added to iSCs, and the iSCs were cultured in the absence of serum (DMEM/F12 F/-/-, 5 × 10⁴ cell/3 cm φ dish) for 4 days. The cultured iSCs were isolated, washed with phosphate buffer, and then lysed in RIPA buffer (4°C, 50 mM Tris-HCl buffer (pH 7.6), 150 mM sodium chloride, 1% Nonidet [registered trademark] P-40 (NP-40), 0.5% sodium deoxycholate, and 0.1% sodium dodecyl sulfate) to obtain homogenates. Concentration of each homogenate was adjusted so that the homogenates had the same total protein content. Then, the homogenate was subjected to SDS-PAGE (BIO-RAD Any kD (trademark)) to separate proteins, and the separated proteins were transferred to polyvinylidene fluoride (PVDF) membrane (Immun-Blot PVDF membrane, Bio-Rad) and blocked by Bloking One (NACALAI TESQUE, INC.). Thereafter, by Western blotting using specific antibody, L-PGDS (anti-Prostaglandin D Synthase (Lipocalin) antibody [EP12357], Abcam, 1 : 2000) and β-Actin (Monoclonal anti-β-Actin [A1978], Sigma, 1 : 100000) were detected. The detection was performed by a high-sensitivity chemiluminescent assay (Chemi-Lumi One L, NACALAI TESQUE, INC.). An example of the results is shown in Fig. 3.

As apparent from Fig. 3, it was confirmed that expression of intracellular L-PGDS protein in iSCs was promoted by the test substance.

### Test Example 3-2: Evaluation of L-PGDS Protein Expression (dot blotting method)

In the presence of a test substance (0, 1, 10, 50 or 100 mNU/mL), iSCs were cultured for 4 days (DMEM/F12 F/-/-, 5 × 10⁴ cell/3 cm φ dish), and 500 µL of the culture supernatant was spotted on a PVDF membrane (Immun-Blot PVDF membrane, Bio-Rad). The membrane was blocked by Bloking One (NACALAI TESQUE, INC.), and L-PGDS was detected by dot blotting using an anti-L-PGDS antibody (anti-Prostaglandin D Synthase (Lipocalin) antibody [EP12357], Abcam, 1 : 2000). An example of the results is shown in Fig. 4.

Since L-PGDS has a typical secretion signal sequence and a typical consensus signal peptidase recognition sequence at the N terminus, it is thought that L-PGDS can be secreted outside from the cell. Thus, L-PGDS protein in the culture supernatant of iSCs to which the test substance was added was examined. As a result, as apparent from Fig. 4, it was confirmed that the amount of L-PGDS protein in the culture supernatant (outside of the cells) was increased depending on dose of the test substance added.

### Test Example 3-3: Evaluation of L-PGDS Protein Expression (ELISA method)

Culture of iSCs (DMEM/F12 F/-/-, 5 × 10⁴ cell/3 cm φ dish) was treated with a test substance (0 [control], 1, 10, 50, 100, or 1000 mNU/mL) for 4 days, and the supernatant was collected. The supernatant was centrifuged (1500 rpm, 10 minutes, 4°C), and then the amount of L-PGDS in the culture supernatant was measured by a specific ELISA method (an ELISA kit for human L-PGDS (Prostaglandin D Synthase 21 kDa (Brain), product number: SEA724Hu, Cloud-Clone Corp.) according to manufacturer's instructions. An example of the results is shown in Table 3 and Fig. 5.

**[Table 3]**

| Additive Concentration of Test Substance (mNU/mL) | Ratio of Expression Level of L-PGDS to Control |
|---|---|
| 0 (Control) | 1.00 |
| 1 | 0.46 |
| 10 | 1.16 |
| 50 | 1.43 |
| 100 | 4.72 |
| 1000 | 6.87 |

As apparent from Table 3 and Fig. 5, and in Test Example 3-2, it was confirmed that when iSCs were treated with the test substance, the amount of L-PGDS protein in the culture supernatant (outside of the cells) was increased. From the results of Test Examples 3, it was found that in iSCs, L-PGDS production was promoted by the test substance at protein level besides gene level, and the produced L-PGDS was secreted.

### Test Example 4: Evaluation of Enzyme Activity of L-PGDS (high performance liquid chromatography (HPLC)-mass spectrometry method)

L-PGDS is an enzyme (EC 5.3.99.2) which catalyzes the biosynthesis of PGD2 from PGH2 as a substrate. In iSCs, for the purpose of confirming that L-PGDS, the production of which has been promoted by a test substance, further produces PGD2 by its enzyme activity, the following analysis was performed. Prostaglandins such as PGD2 and its metabolites, and PGE2 produced from PGH2, which is a common substrate of prostaglandin synthesis, were comprehensively analyzed concerning: (A) inside of the cells and (B) outside of the cells of iSCs.

### A.: Evaluation of Enzyme Activity concerning Inside of Cells of iSCs

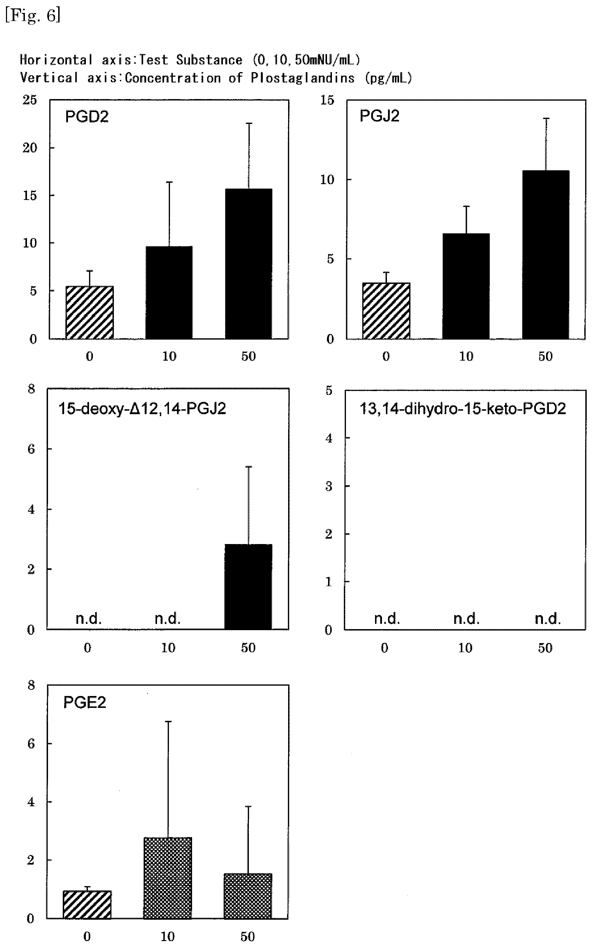

A test substance (0, 10, or 50 mNU/mL) was added to iSCs, the iSCs were cultured for 3 days (FBS-free DMEM/F12 F/-/-, 5 × 10⁴ cell/3 cm φ dish), and a cell extract was prepared from the cultured iSCs by RIPA buffer treatment (4°C, 20 min). Each of the concentrations of prostaglandins (PGD2, PGJ2, 15-deoxy-Δ12,14-PGJ2, 13,14-dihydro-15-keto-PGD2, and PGE2) in the cell extract was measured by HPLC-mass spectrometry. For HPLC separation, AQUITY UPLC HSS T3 column (Waters) was used. As a detector and a mass spectrometer, API 4000 LC/MS/MS system and Triple Quadrupole (both from AB Sciex) were used, respectively. An example of the results is shown in Table 4 and Fig. 6.

**[Table 4]**

| (n=3)Additive Concentration of Test Substance (mNU/mL) | Concentration of prostaglandins (pg/mL) | | | | |
|---|---|---|---|---|---|
| | PGD2 | PGJ2 | 15- deoxy - Δ12,14-PGJ2 | 13,14-dihydro-15-keto -PGD2 | PGE2 |
| 0 | 5.45±1.62 | 3.47±0.68 | n.d. | n.d. | 0.93±0.16 |
| 10 | 9.63±6.75 | 6.59±1.71 | n.d. | n.d. | 2.76±3.98 |
| 1000 | 15.67±6.89 | 10.57±3.28 | 2.83±5.58 | n.d. | 1.52±2.32 |

As shown in Table 4 and Fig. 6, intracellular level of PGD2 and PGJ2, which is a nonenzymatic metabolite of PGD2, were increased by the addition of the test substance in a dose dependent manner. Further, when 50 mNU/mL of the test substance was added, production of 15-deoxy-Δ12,14-PGJ2, which is a nonenzymatic metabolite of PGJ2, was also observed. The amount of 13,14-dihydro-15-keto-PGD2, which is a nonenzymatic metabolite of 15-deoxy-Δ12,14-PGJ2, was not greater than the detection limit. On the other hand, with respect to PGE2 produced from PGH2, which is a substrate for PGE2 as well as PGD2, no production response depending on dose of the test substance added was observed.

### B.: Evaluation of Enzyme Activity concerning Outside of Cells of iSCs

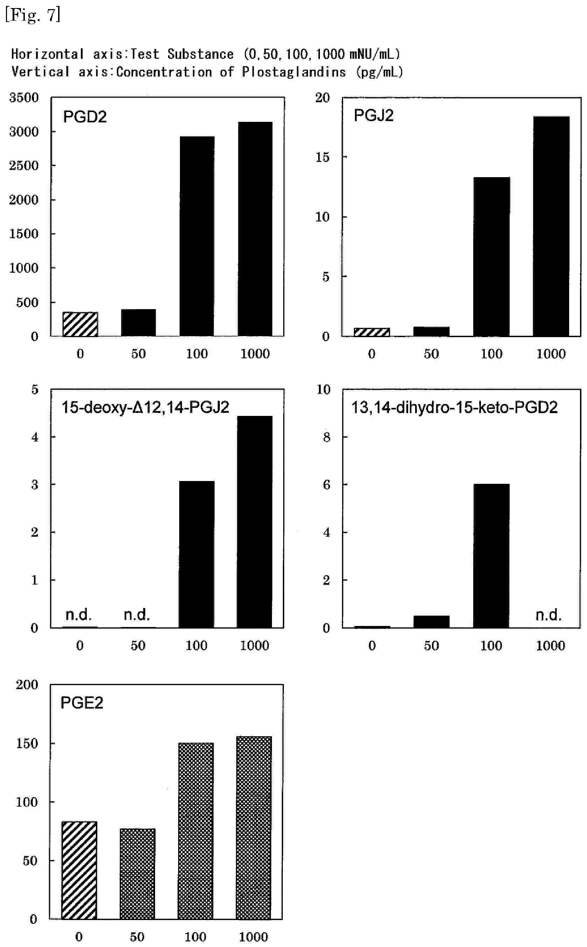

A test substance (0, 50, 100, or 1000 mNU/mL) was added to iSCs, and the iSCs were cultured for 3 days (FBS-free DMEM/F12 F/-/-, 5 × 10⁴ cell/3 cm φ dish). The culture supernatant was reacted with PGH2, which is a substrate of L-PGDS, and glutathione (GSH) (reaction condition: 100 mM Tris-HCl (pH 8.0), 1 mM GSH, 10 mM PGH2, 37°C, 5 min). As in the above-described A., each of the concentrations of prostaglandins (PGD2, PGJ2, 15-deoxy-Δ12,14-PGJ2, 13,14-dihydro-15-keto-PGD2, and PGE2) in the reaction solution was measured by HPLC-mass spectrometry. An example of the results is shown in Table 5 and Fig. 7.

**[Table 5]**

| (n=1) | | | | | |
|---|---|---|---|---|---|
| Additive Concentration of Test Substance (mNU/mL) | Concentration of prostaglandins (pg/mL) | | | | |
| | PGD2 | PGJ2 | 15- deoxy - Δ12,14-PGJ2 | 13,14-dihydro-15-keto -PGD2 | PGE2 |
| 0 | 351 | 0.7 | n.d. | 0.061 | 83.0 |
| 50 | 395 | 0.8 | n.d. | 0.491 | 76.9 |
| 100 | 2928 | 13.3 | 3.1 | 6.002 | 150.5 |
| 1000 | 3139 | 18.4 | 4.4 | n.d. | 155.6 |

As shown in Table 5 and Fig. 7, concentrations of PGD2 and PGJ2, which is a nonenzymatic metabolite of PGD2, and 15-deoxy-Δ12,14-PGJ2 were increased by the test substance at concentrations of 100 mNU/mL or higher. Thus, it was confirmed that L-PGDS activity was present in iSC culture supernatant. On the other hand, dose-dependent production of 13,14-dihydro-15-keto-PGD2, which is a nonenzymatic metabolite of 15-deoxy-Δ12,14-PGJ2, was observed by the addition of the test substance at concentrations up to 100 mNU/mL. However, with 1000 mNU/mL of the test substance, the amount of 13,14-dihydro-15-keto-PGD2 was lower than the detection limit.

It is known that L-PGDS is a secretory enzyme. From the results of the above-described A. and B., it was confirmed that iSCs secreted L-PGDS having an enzyme activity into the outside of the cells, and the production and secretion of L-PGDS were promoted by the addition of the test substance.

### Test Example 5-1: Examination of L-PGDS-producing Cells in Mouse Brain (immunohistochemical staining method)

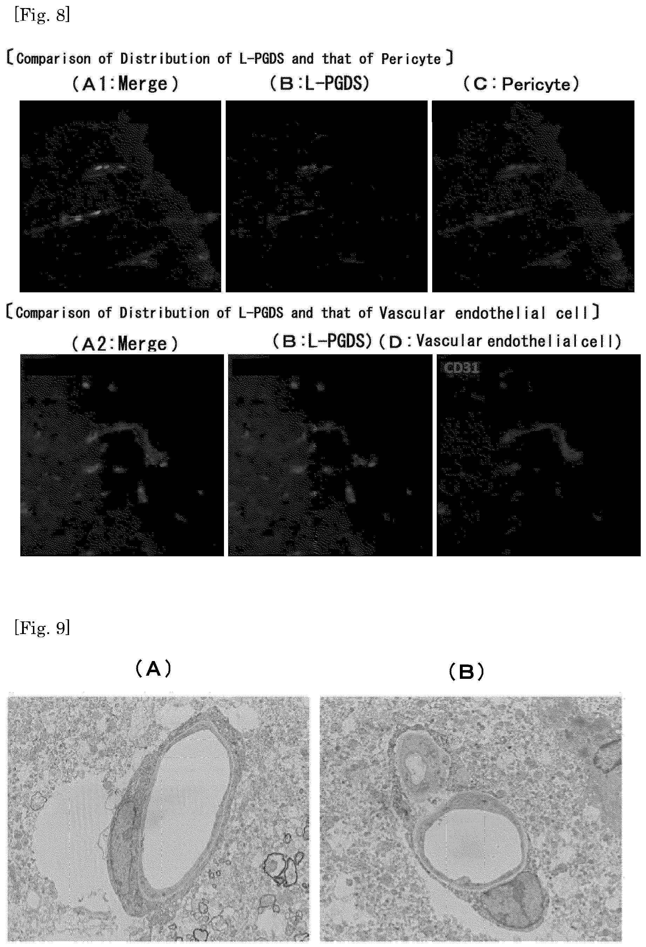

Ischemic insult was applied to a C.B-17 mouse by middle cerebral artery occlusion. At three days after the application of ischemic insult, brain slices were prepared, and immunohistochemical examination was performed with a confocal laser microscope using specific antibodies against L-PGDS (panel B, green; anti-prostaglandin D synthase (lipocalin) antibody [EP12357], Abcam, 1 : 1000), a pericyte marker α-SMA (panel C, red; anti-actin, smooth muscle, clone ASM-1, Millipore, 1 : 1000), and a vascular endothelial cell marker CD31 (panel D, red; anti-mouse CD31 (PECAM-1) monoclonal antibody, 550274, BD Pharmingen, 1 : 1000). By multiple staining, L-PGDS (panel B) and the pericyte marker (panel C), or L-PGDS (panel B) and the vascular endothelial cell marker (panel D) were detected using specific antibodies each labeled with different fluorescent dyes, and images were obtained. The images were merged, and distribution was compared between L-PGDS and the pericyte marker, or L-PGDS and the vascular endothelial cell marker (panel A1, panel A2). An example of the results is shown in Fig. 8.

In images in the upper half of Fig. 8, green fluorescence in panel B represents distribution of L-PGDS, and red fluorescence in panel C represents distribution of the pericyte marker. In panel A1 in which these two images are merged, L-PGDS (green fluorescence) partially overlaps with the distribution of the pericyte marker (red fluorescence) (magnification of ×200). On the other hand, in images in the lower half of Fig. 8, green fluorescence in panel B represents distribution of L-PGDS, and red fluorescence in panel D represents distribution of the vascular endothelial cell marker. In panel A2 in which these two images are merged, although distribution of L-PGDS (green fluorescence) is close to that of the vascular endothelial cell marker (red fluorescence), no image showing co-distribution was obtained (magnification of ×200).

L-PGDS is a protein mainly distributed in the central nervous system, exists in the arachnoid membrane and the pia mater, the choroid plexus of the lateral ventricles of the brain, and the like, and it is thought that L-PGDS is secreted into cerebrospinal fluid (Urade Y. et al., J Lipid Mediat. Cell Signal. 14, 71-82, 1996). However, L-PGDS-producing cells in the brain have not yet sufficiently been elucidated. The present inventors have made investigations of distribution of L-PGDS in the brain using an immunohistochemical method. As a result, it was found that the expression of L-PGDS in the normal brain of a C.B-17 mouse was weak. In the above-described Test Example 4-1, it was also found that when permanent ligation was performed on a middle cerebral artery of the mouse, a significant L-PGDS expression was observed in the cerebral infarction area (panel B of Fig. 8). In addition, although distribution of L-PGDS was observed around the vascular endothelial cell marker CD31, the distribution of L-PGDS and the distribution of CD31 did not overlap with each other (panel A2 of Fig. 8). On the other hand, it was found that L-PGDS was partially co-localized with a pericyte marker α-SMA (panel A1 of Fig. 8). From the above results, it was thought that L-PGDS, the expression of which was promoted in a cerebral infarction area, was derived from pericytes (or iSCs). This was also confirmed from the following results, which were obtained by immunoelectron microscopy in Test Example 4-2, that L-PGDS-positive product was observed as an electron-dense structure in the cytoplasm of pericytes, which were present in contact with vascular endothelial cells and the basal membrane (panels A and B of Fig. 9). From these results, it was supposed that the promotion of L-PGDS expression was included in physiological roles of pericytes or iSCs in ischemic response.

Test Example 5-2: Examination of L-PGDS-producing Cells in Mouse Brain (immunoelectron microscopy)

Ischemic insult was applied to a C.B-17 mouse by middle cerebral artery occlusion. At three days after the application of ischemic insult, brain slices were prepared, and L-PGDS expression sites were observed by immunoelectron microscopy using a specific antibody against L-PGDS (anti-prostaglandin D synthase (lipocalin) antibody [EP12357], Abcam, 1 : 1000). Specifically, the observation was carried out as follows. Brain slices each having a thickness of 2 µm were prepared using a vibratome. The slices were subjected to a reaction using an avidin-biotin horseradish peroxidase (HRP) complex kit (Vector Laboratories) and 3,3'-diaminobenzidinetetrahydrochloride (DAB), treated with osmium, and embedded in epon. Then, ultrathin slices were prepared from the epon-embedded slice, and the ultrathin slices were observed by electronmicroscopy. An example of the results is shown in Fig. 9.

In both panels A and B of Fig. 9, by immunoelectron microscopic observation about L-PGDS, electron-dense regions were observed in the cytoplasm of vascular perivascular cells (pericytes), which were present in contact with vascular endothelial cells and the basal membrane.

### Test Example 6: Examination of L-PGDS-producing Cells in Human Brain (immunohistochemical staining method)

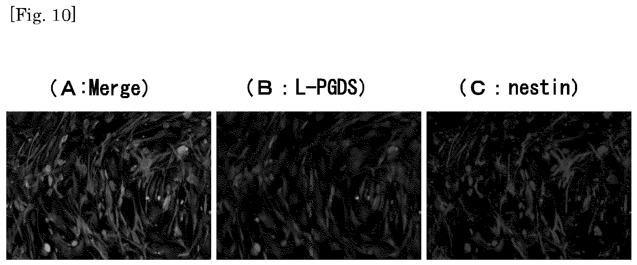

According to a method described in Tatebayashi K. et al., Stem Cells and Develop. 26, 787-797, 2017, immunohistochemical investigation of cultured iSCs isolated from a human cerebral infarction area (necrotic tissue) was performed using specific antibodies against L-PGDS (panel B, green; anti-prostaglandin D synthase (lipocalin) antibody [EP12357], Abcam, 1 : 1000) and nestin, which is a neural stem cell marker, (panel C, red; anti-nestin, clone 10C2, Millipore, 1 : 1000). The cells were reacted with an Alexa Fluor [registered trademark, the same applies hereinafter] 488-conjugated antibody or an Alexa Fluor 555-conjugated antibody (1 : 500; Molecular Probes, Eugene), and thereafter subjected to nuclear staining with 4',6-diamidino-2-phenylindole (DAPI; 1 : 1000; Kirkegaard & Perry Laboratories). Fluorescence imaging of the stained cells were performed using a fluorescent microscope (BX60; Olympus, Japan), images of L-PGDS (panel B) and nestin (panel C) were merged, and distributions of L-PGDS and nestin were compared with each other (panel A). An example of the results is shown in Fig. 10.

In Fig. 10, green fluorescence in panel B represents distribution of L-PGDS, and red fluorescence in panel C represents distribution of nestin. In panel A in which these two images are merged, L-PGDS (green fluorescence) overlaps with the distribution of nestin (red fluorescence) (magnification of ×200). That is, it was confirmed that L-PGDS was expressed in almost all nestin-expressing iSCs. From these results, it was thought that L-PGDS was produced in iSCs or pericytes not only in the mouse brain but also in the human brain.

### Test Example 7: Evaluation of Cerebral Amyloid β Deposition and L-PGDS Expression in Alzheimer-type Dementia Model Animal

### (1) Behavioral Analysis of Cognitive Ability and Sample Preparation

APPswe/PS1dE9 (APP/PS1) mice (female, 3-month old) were divided into a test substance-administration group and a control group (10 to 11 animals/group) such that each group had the same body weight. The test substance (100 NU/kg body weight) and physiological saline were administered to the animals of the test substance-administration group and the animals of the control group, respectively, twice a week for about 3 months by tail vein injection. After the final administration, behavioral analyses of cognitive ability (a Y-maze test, a novel object recognition test, and the Morris water maze test) were performed. In each of the tests, each of the following A to C was measured. In the Y-maze test, (A) a rate of alternation behavior (%) was measured. In the novel object recognition test, (B) a frequency of access to a novel object (%) was measured. In the Morris water maze test, (C) an average time (seconds) required for finding the platform during 4 days until the end of the learning period was measured (from day 6 to day 9). Then, a composite score (A × B ÷ C) was calculated for each individual animal. The evaluation results in two individuals of each group are shown in Table 6.

**[Table 6]**

| | Individual Number | Y-maze Test A (%) | Novel Object Recognition Test B (%) | Morris Water Maze Test C (Sec.) | Composite Score (A×B÷C) |
|---|---|---|---|---|---|
| Control Group | #14 | 36.8 | 57.1 | 84.4 | 25.0 |
| | #30 | 40.0 | 47.1 | 90.0 | 20.9 |
| Test Substance-Administration Group | #4 | 52.6 | 66.7 | 48.9 | 71.8 |
| | #29 | 61.5 | 60.0 | 40.0 | 92.3 |

As apparent from Table 6, an improvement action on cognitive ability was observed in the test substance-administration group, and the difference in this action was statistically significant between the two groups.

After the behavioral analysis of cognitive ability (24 hours after the final administration), brains were harvested from animals of the test substance-administration group and the control group by decapitation under anesthesia, and the left hemisphere of the brain was quickly frozen in a liquid nitrogen bath. On the other hand, the remaining right hemisphere was fixed (at 4°C) with (A) a fixative solution containing glutaraldehyde (0.05% glutaraldehyde, 4% paraformaldehyde, and 0.1 M phosphate buffer) or (B) a PLP fixative solution (0.01 M sodium metaperiodate, 0.075 M lysine, and 2% paraformaldehyde). From the fixed tissue sample, a coronal plane slices (thickness: 20 µm) including the cerebral cortex was prepared using a cryostat (Leica CM1850).

### (2) Evaluation of Amyloid β Deposition in Cerebral Cortex (immunohistochemical staining method)

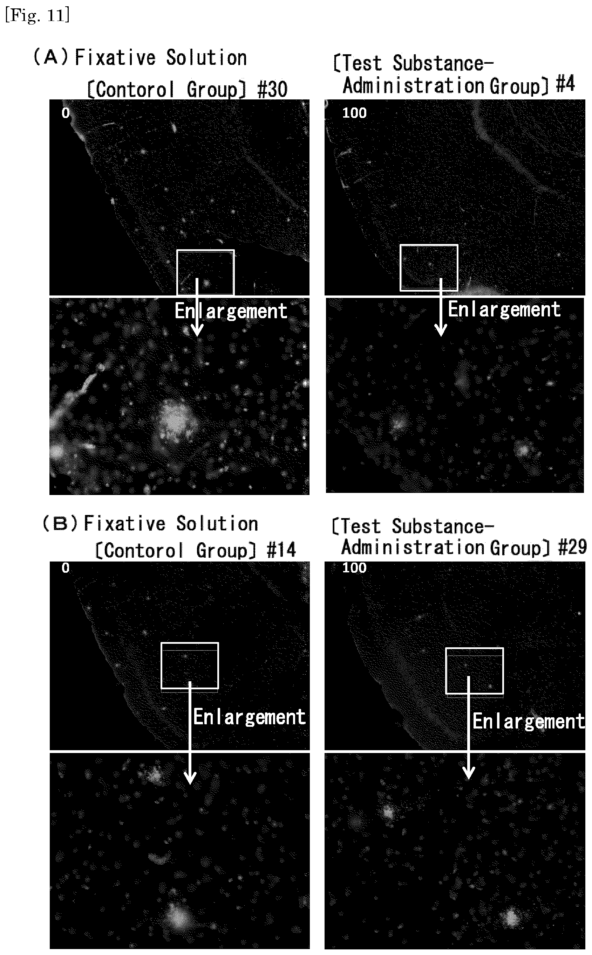

The coronal plane slices of the test substance-administration group and the control group prepared in the above-described (1) were immunohistochemically stained with an anti-amyloid β antibody (anti-β-amyloid, 1-16, [SIG-39300], BioLegend, 1 : 1000). The anti-amyloid β antibody-stained slices were reacted with an Alexa Fluor 488-conjugated antibody (1 : 500; Molecular Probes, Eugene), thereafter nuclear staining was performed with 4',6-diamidino-2-phenylindole (DAPI; 1 : 1000; Kirkegaard & Perry Laboratories), and fluorescence imaging of amyloid β was performed using a fluorescent microscope (BX60; Olympus, Japan). An example of the results is shown in Fig. 11.

As shown in Fig. 11, in individuals that were improved in cognitive ability by the administration of the test substance, the number and the area of amyloid plaques in the brain were decreased as compared to the control group.

### (3) Evaluation of L-PGDS Expression in Cerebral Cortex (immunohistochemical staining method)

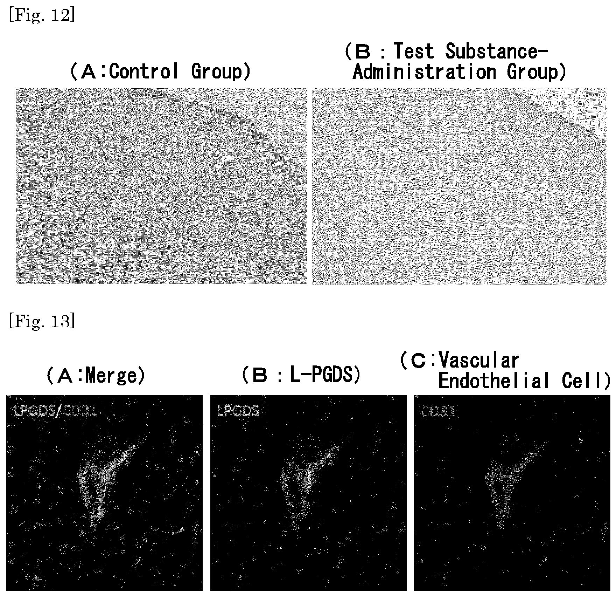

The coronal plane slices of the test substance-administration group and the control group prepared in the above-described (1) were immunohistochemically stained with an anti-L-PGDS antibody (anti-prostaglandin D synthase (lipocalin) antibody [EP12357], Abcam, 1 : 1000). L-PGDS was detected by the 3,3'-diaminobenzidine tetrahydrochloride (DAB) reaction using an avidin-biotin horseradish peroxidase (HRP) complex kit (Vector Laboratories). An example of the results is shown in Fig. 12.

As shown in Fig. 12, in the brain of the test substance-administration group, specific staining of L-PGDS was observed in the vicinity of vascular cavities (indicated by the arrow in panel B). On the other hand, in the brain of the control group, no specific staining of L-PGDS was observed (panel A).

With respect to the coronal plane slices of the test substance-administration group prepared in the above-described (1), immunohistochemical examination was performed using specific antibodies against L-PGDS (anti-prostaglandin D synthase (lipocalin) antibody [EP12357], Abcam, 1 : 1000) and a vascular endothelial cell marker CD31 (anti-mouse CD31 (PECAM-1) monoclonal antibody, 550274, BD Pharmingen, 1 : 1000). A slice to which the above-described primary antibodies were bound was reacted with an Alexa Fluor 488-conjugated antibody or an Alexa Fluor 555-conjugated antibody (1 : 500; Molecular Probes, Eugene), and thereafter subjected to nuclear staining with 4',6-diamidino-2-phenylindole (DAPI; 1 : 000; Kirkegaard & Perry Laboratories). Fluorescence imaging of the stained slice was performed using a confocal laser microscope (LSM780; Carl Zeiss Jena, Germany). By the multiple staining, images of L-PGDS (panel B) and CD31 (panel C) were merged, and distribution was compared between L-PGDS and CD31 (panel A). An example of the results is shown in Fig. 13.

As shown in Fig. 13, in the brain of the test substance-administration group, it was observed that L-PGDS (green fluorescence) was distributed around the vascular endothelial cell marker CD31 (red fluorescence). From the results, it was thought that in response to the administration of the test substance, the expression of L-PGDS was increased in pericytes (or iSCs) which were present around vascular endothelial cells.

### (4) Evaluation of Cerebral Amyloid β Deposition and L-PGDS Expression (western blotting method)

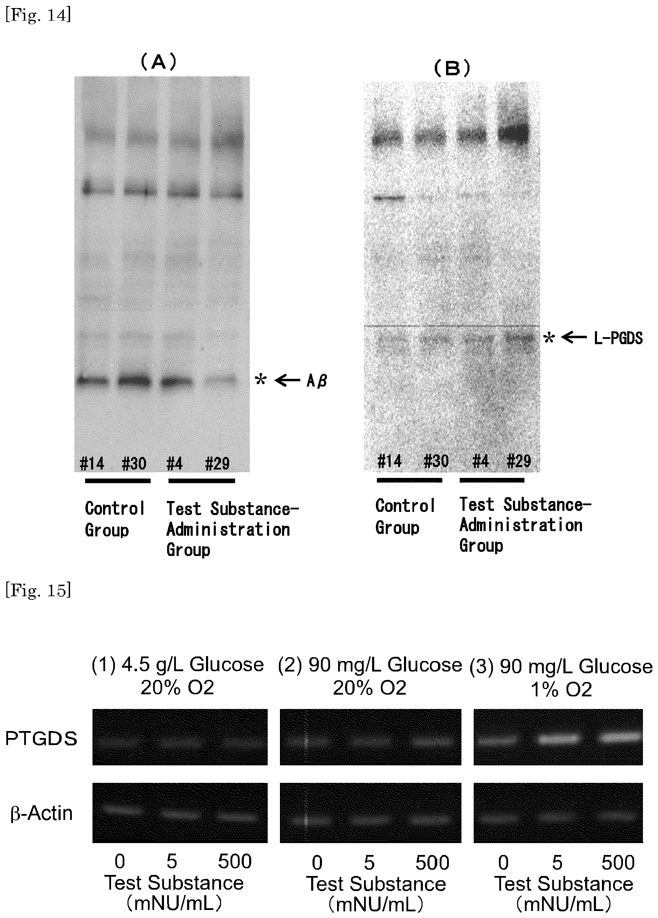

The left hemisphere of the brain harvested and quickly frozen in the above-described (1) was homogenized using Potter-Elvehjem homogenizer to extract proteins. The extracted proteins were separated by SDS-PAGE (BIO-RAD Any kD (trademark)), and transferred to a PVDF membrane (Immun-Blot PVDF membrane, Bio-Rad). The PVDF membrane was blocked with Bloking One (NACALAI TESQUE, INC.). Then, amyloid β (anti-β-amyloid, 1-16, [SIG-39300], BioLegend, 1 : 1000) and L-PGDS (anti-Prostaglandin D Synthase (Lipocalin) antibody [EP 123571, Abcam, 1 : 2000) were detected by Western blotting using specific antibodies. The detection was performed by a high-sensitivity chemiluminescent assay (Chemi-Lumi One L, NACALAI TESQUE, INC.). An example of the results is shown in Fig. 14.

As shown in the position indicated by the symbol * in panel A of Fig. 14, in the mice in the test substance-administration group (individual numbers: 4 and 29) which were improved in cognitive ability by the administration of the test substance, the amount of amyloid β was reduced as compared to the mice in the control group (individual numbers: 14 and 30). The results were correlated with the results of the immunohistochemical staining in Test Example 7 (2) (Fig. 11) that the number and the area of cerebral amyloid β antibody-positive product were reduced by test substance administration as compared to the control group. In addition, as shown in the position indicated by the symbol * in panel B of Fig. 14, in the test substance-administration group, the amount of intracerebral L-PGDS was increased as compared to the control group, and therefore it was confirmed that intracerebral L-PGDS production was promoted by the test substance. From the above-described results in Test Example 7, it is thought that it may be possible that the test substance promotes the expression of L-PGDS in the brain, leading to prevention of cerebral amyloid β deposition, which in turn improve cognitive function.

Test Example 8: Examination of Contributing Factor for Promotion of L-PGDS Expression in Human-derived Pericytes (classical RT-PCR method)

As described above, it is thought that iSCs that respond to the test substance are derived from brain pericytes. Thus, using commercially available Human Brain Vascular Pericytes (ScienCell), which is a human pericyte cell line, under different oxygen and/or glucose concentrations, L-PGDS expression activity of the test substance was examined. Specifically, in the presence of different doses, which were 0, 50, or 500 mNU/mL, of the test substances, iSCs were cultured for 4 days (FBS-free DMEM medium F//-) under the following conditions: (1) a condition in which iSCs show reactivity (4.5 g/L of glucose and 20% of O₂), (2) a low glucose condition (90 mg/L of glucose and 20% of O₂), and (3) a low oxygen and low glucose condition (90 mg/L of glucose and 1% of O₂). From the cultured iSCs, total RNA was extracted using RNeasy Mini Kit (QIAGEN), and L-PGDS gene (PTGDS) expression level was semiquantified by classical RT-PCR method using SuperScript III One-Step RT-PCR System with Platinum (Invitrogen) (35 cycles). The PCR products were separated by 2% agarose gel electrophoresis, and bands of PTGDS and β-Actin were stained with ethidium bromide and visually detected. The primers used for PTGDS and β-Actin were same as used in Test Example 2-1. An example of the results is shown in Fig. 15.

As shown in Fig. 15, although the transferred L-PGDS gene of the cells cultured under condition (1) was capable of being detected, no responsivity to the test substance was observed. On the other hand, in the cells cultured under condition (3), which is a low oxygen (1%) and low glucose concentration (90 mg/L) condition, a remarkable responsivity to the test substance was observed. It is thought that the low oxygen and low glucose condition mimics the ischemic condition in the brain. Thus, it is thought that under conditions of ischemic diseases, pericytes (or iSCs) acquire responsivity to an external stimulus, and the pericytes (or iSCs) release cyto-protective proteins such as L-PGDS.

### [Industrial Applicability]

It is said that L-PGDS is a protein that is mainly expressed in the brain, plays roles as a binder and transporter or a scavenger for various hydrophobic low-molecular weight compounds, and has various functions such as an intracerebral environment-controlling and brain-protective function, and a sleep-controlling function. Thus, the L-PGDS production promoting agent is useful as a preventive, therapeutic or relapse preventive agent for L-PGDS associated diseases including a cerebrovascular disorder such as cerebral infarction, dementia such as Alzheimer's disease, and insomnia. In particular, the present extract and a preparation containing the present extract are highly useful as excellent L-PGDS production promoting agents and highly safe drugs with little problem such as side effects. In addition, a screening method of the present invention as defined in the appended claims for a substance useful for prevention/treatment or relapse prevention of L-PGDS associated diseases , in particular, a substance having a brain-protective action or a sleep-promoting action, using an L-PGDS production promoting action in pericytes or iSCs dedifferentiated from pericytes as an index is an extremely useful method that contributes to the development of a new therapeutic agent.

**[Sequence Listing]**

## Claims

1. A screening method for a substance having a brain-protective action or a sleep-promoting action, comprising the steps of screening substances using a lipocalin-type prostaglandin D2 synthase expression-promoting action in pericytes or ischemia-induced multipotent stem cells dedifferentiated from pericytes as an index, and selecting a substance as having a brain-protective action or a sleep-promoting action if the substance promotes the lipocalin-type prostaglandin D2 synthase expression.

2. The screening method according to claim 1, wherein the substance having a brain-protective action is for prevention, treatment or relapse prevention of a cerebrovascular disorder, or for prevention or treatment of dementia.

3. A determining or evaluating method for an extract from inflamed skin tissues of rabbits inoculated with vaccinia virus or a preparation containing the extract, comprising the steps of providing an extract from inflamed skin tissues of rabbits inoculated with vaccinia virus or a preparation containing the extract and determining or evaluating that the extract or the preparation is for the protection of brain or the sleep promotion if it promotes lipocalin-type prostaglandin D2 synthase expression, optionally, if it promotes lipocalin-type prostaglandin D2 synthase expression in pericytes or ischemia-induced multipotent stem cells dedifferentiated from pericytes.

4. A method for verifying that an extract from inflamed skin tissues of rabbits inoculated with vaccinia virus or a preparation containing the extract satisfies a quality standard by performing the determination or evaluation as described in claim 3.

## Patentansprüche

1. Screeningverfahren nach einer Substanz, die eine schützende Wirkung auf das Gehirn oder eine schlaffördernde Wirkung hat, umfassend die Schritte des Screenings von Substanzen unter Verwendung einer expressionsfördernde Wirkung einer Lipocalin-Typ-Prostaglandin-D2-Synthase in Perizyten oder ischämieinduzierten multipotenten Stammzellen, die aus Perizyten dedifferenziert wurden, als einen Index, und Auswählen einer Substanz als eine schützende Wirkung auf das Gehirn oder eine schlaffördernde Wirkung habend, wenn die Substanz die Expression von Lipocalin-Typ-Prostaglandin-D2-Synthase fördert.

2. Screeningverfahren gemäß Anspruch 1, wobei die Substanz, die eine schützende Wirkung auf das Gehirn hat, zur Vorbeugung, Behandlung oder Rückfallprävention einer zerebrovaskulären Erkrankung oder zur Vorbeugung oder Behandlung von Demenz ist.

3. Bestimmungs- oder Bewertungsverfahren für einen Extrakt aus entzündetem Hautgewebe von Kaninchen, die mit Vacciniavirus inokuliert wurden, oder ein Präparat, das den Extrakt enthält, umfassend die Schritte des Bereitstellens eines Extrakts aus entzündetem Hautgewebe von Kaninchen, die mit Vacciniavirus inokuliert wurden, oder eines Präparats, das den Extrakt enthält, und des Bestimmens oder Bewertens, dass der Extrakt oder das Präparat zum Schutz des Gehirns oder zur Förderung des Schlafes geeignet ist, wenn er die Expression von Lipocalin-Typ-Prostaglandin-D2-Synthase fördert, optional, wenn er die Expression von Lipocalin-Typ-Prostaglandin-D2-Synthase in Perizyten oder ischämieinduzierten multipotenten Stammzellen, die aus Perizyten dedifferenziert wurden, fördert.

4. Verfahren zum Verifizieren, dass ein Extrakt aus entzündetem Hautgewebe von Kaninchen, die mit dem Vacciniavirus inokuliert wurden oder ein den Extrakt enthaltendes Präparat, einen Qualitätsstandard erfüllt, indem die in Anspruch 3 beschriebene Bestimmung oder Bewertung durchgeführt wird.

## Revendications

1. Procédé de criblage pour une substance présentant une action neuroprotectrice ou une action favorisant le sommeil, comprenant les étapes de criblage de substances utilisant une action favorisant l'expression de la prostaglandine D2 synthase de type lipocaline dans les péricytes ou les cellules souches multipotentes induites par ischémie dédifférenciées à partir de péricytes comme indice, et de sélection d'une substance comme présentant une action neuroprotectrice ou une action favorisant le sommeil si la substance favorise l'expression de la prostaglandine D2 synthase de type lipocaline.

2. Procédé de criblage selon la revendication 1, dans lequel la substance présentant une action neuroprotectrice est destinée à la prévention, au traitement ou à la prévention de la rechute d'un trouble cérébrovasculaire, ou à la prévention ou au traitement de la démence.

3. Procédé de détermination ou d'évaluation pour un extrait de tissus cutanés enflammés de lapins inoculés avec un virus vaccinal ou une préparation contenant l'extrait, comprenant les étapes de fourniture d'un extrait de tissus cutanés enflammés de lapins inoculés avec un virus vaccinal ou d'une préparation contenant l'extrait et de détermination ou d'évaluation que l'extrait ou la préparation est destiné à la protection du cerveau ou à la promotion du sommeil s'il favorise l'expression de la prostaglandine D2 synthase de type lipocaline, éventuellement, s'il favorise l'expression de la prostaglandine D2 synthase de type lipocaline dans les péricytes ou les cellules souches multipotentes induites par ischémie dédifférenciées à partir de péricytes.

4. Procédé pour vérifier qu'un extrait de tissus cutanés enflammés de lapins inoculés avec un virus vaccinal ou une préparation contenant l'extrait satisfait à une norme de qualité en effectuant la détermination ou l'évaluation telle que décrite dans la revendication 3.
